# EUROPÄISCHE PATENTSCHRIFT

(11) **EP 0 600 571 B1**
(45) Veröffentlichungstag und Bekanntmachung des Hinweises auf die Patenterteilung: **02.05.2002**
(21) Anmeldenummer: 93250333.7
(22) Anmeldetag: 02.12.1993
(51) Int. Cl.: G01S 13/56, G01S 7/02, G01S 7/03

(54) **Elektronisches Lebensdetektionssystem**
Electronic life detection system
Système électronique pour la détection de vivants

(30) Priorität: 04.12.1992 DE 4241664
(43) Veröffentlichungstag der Anmeldung: 08.06.1994
(73) Patentinhaber: BORUS SPEZIALVERFAHREN UND- GERÄTE IM SONDERMASCHINENBAU GmbH, D-12459 Berlin (DE)
(72) Erfinder: Hablov, Dimitri Vladimivovich, 119034 Moskau (RU); Fisun, Oleg Ivanovich, 119034 Moskau (RU); Lupichev, Lev Nikolaevich, 119034 Moskau (RU); Osipov, Viktor Vrastislavovich, 119034 Moskau (RU); Schestiperov, Viktor Alexandrovich, 119034 Moskau (RU); Schimko, Richard, D-10179 Berlin (DE)
(74) Vertreter: Eisenführ, Speiser & Partner

(56) Entgegenhaltungen:
- EP-A- 0 188 757
- GB-A- 2 191 358
- US-A- 4 490 718
- IEEE TRANSACTIONS ON BIO-MEDICAL ENGINEERING Bd. BME-33, Nr. 7 , Juli 1986 , NEW YORK US Seiten 697 - 700 CHEN ET AL. 'An X-Band Microwave Life-Detection System'
- 1990 INTERNATIONAL SYMPOSIUM DIGEST ANTENNAS AND PROPAGATION Bd. 2 , 7. Mai 1990 , DALLAS, USA Seiten 738 - 741 XP167387 YAMAGUCHI ET AL. 'FM-CW Radar applied to the Detection of buried Objects in Snowpack'

## Beschreibung

Die Erfindung betrifft ein elektronisches Lebensdetektionssystem der im Oberbegriff des Anspruchs 1 angegebenen Art.

Es ist bekannt, das von einer Person ausgehende Wellenfeld in analoger Weise als Nachweisfeld zu nutzen.

In DE 38 32 428 A1 ist beispielsweise ein solches elektronische Überwachungssystem auf der Grundlage der von Personen ausgehenden Infrarotstrahlung beschrieben.

Schließlich ist auch die Nutzung von Mikrowellenstrahlung zur Bewegungsdetektion in offenem Gelände oder innerhalb von Räumen bekannt, die auf der Modulation eines Mikrowellenstrahles durch sich im der Strahlung ausgesetzten Bereich bewegende Objekte beruht.

Es sind weiter Suchvorrichtungen bekannt, die mit Mikrowellen arbeiten und zum Auffinden von durch Erdbeben oder Lawinen von der Außenwelt abgeschnittenen Personen eingesetzt werden.

Die Verwendung von Mikrowellen zum Auffinden verschütteter Personen basiert auf dem Umstand, daß Mikrowellen zum Teil in der Lage sind, Schutt- und Geröllmaterialien zu durchdringen und daß die reflektierten Anteile je nach Materie unterschiedliche Eigenschaften zeigen.

Aus K. Chen et al. "An X-band microwave life-detection system", IEEE Transactions on biomedical engineering, Vol. BME 33, No. 7, July 1986 ist ein derartiger mit Mikrowellen arbeitender elektronischer Lebensdetektor nach dem Oberbegriff des Patentanspruchs 1 bekannt.

Bei diesem elektronischen Lebensdetektor werden Mikrowellen einer bestimmten Frequenz kontinuierlich in einen zu untersuchenden räumlichen Bereich eingestrahlt.

Das von dem abgetasteten Bereich reflektierte Signal weist, wenn sich darin ein lebender Mensch befindet, einen unmodulierten und einen durch die Körperfunktionen, speziell Atmung und Herzschlag, des von der Außenwelt abgeschnittenen Menschen modulierten Anteil auf. Durch eine in Phase und Dämpfungsmaß regelbare Kompensationsschleife wird der nichtmodulierte Anteil des reflektierten Signals gelöscht.

Der Informationsinhalt des modulierten Anteils des reflektierten Signals wird also durch einen Phasenvergleich mit dem ausgesandten (Abtast-)Signal selektiert.

In der genannten Druckschrift wird auch eine zur berührungslosen Patientenüberwachung geeignete Abwabdlung des Verfahrens beschrieben, die ohne Richtantenne und mit 0,1 mW Ausgangsleistung auskommt.

Diese bekannte Vorrichtung ist jedoch für einen Einsatz unter ungünstigen Bedingungen, bei denen die empfangenen Reflexionssignale sehr schwach und/oder in ihrer Intensität stark schwankend sind, sowie insbesondere für eine präventive Überwachung von Gebäuden nur bedingt geeignet.

Ferner ist US-Patentschrift 4 967 751 eine Vorrichtung bekannt, bei der die einen lebenden Körper durchstrahlenden Mikrowellen einer Frequenzanalyse unterzogen werden. Dieses Verfahren ist jedoch nicht zur Detektion von Lebewesen geeignet, da ein Gebäude oder ein Verschüttungsbereich üblicherweise nicht von einander gegenüberliegenden Seiten zugänglich ist.

Die GB 21 91 358 beschreibt ein Dopplerradar bei 24 GHz, welches die durch die Bewegung der Objekte entstehenden Frequenzen auswertet, da die Ortsveränderung von ihren Ort im Raum ändernden Personen oder Objekten vom Auftreten eines Dopplereffektes begleitet ist.

Die US 4,490,718 beschreibt ein Radargerät zur Auswertung von Vibrationsspektren von laufenden Maschinen, Motoren usw. auf bewegten oder unbewegten Zielen durch Vergleiche mit bekannten bzw. durch Neuklassifizierung von unbekannten Spektren. Es werden hier Sendefrequenzen zwischen 8 und 12 GHz verwendet.

Der Erfindung liegt daher die Aufgabe zugrunde, ein elektronisches Lebensdetektionssystem der eingangs genannten Gattung zu schaffen, das sich durch erweiterte Einsatzmöglichkeiten unter ungünstigen Bedingungen und insbesondere zur Überwachung von Gebäuden eignet.

Diese Aufgabe wird durch ein elektronisches Überwachungssystem mit den Merkmalen des Anspruches 1 gelöst.

Praktische Ergebnisse haben gezeigt, daß überraschend in einem Frequenzbereich von 1,3 bis 1,6 GHz die zur Messung von Körpersignalen durch bauliche Umhüllungen hindurch erforderliche Mikrowellenleistung besonders gering ist, so daß sich dieser Frequenzbereich für ein Überwachungssystem besonders gut eignet.

Die Erfindung schließt die Erkenntnis ein, daß die Anwesenheit bzw. Identität von Personen oder anderen Lebewesen aufgrund des modulierten Anteils des reflektierten Mikrowellensignals erkennbar ist, wenn dieses einer Frequenzanalyse unterzogen wird. Das Frequenzspektrum bildet eine Art "elektronischen Fingerabdruck" des Lebewesens mit charakteristischen Merkmalen, welcher einerseits eine Erkennung durch Vergleich mit gespeicherten Mustern, andererseits aber auch eine Unterscheidung verschiedener Lebewesen ermöglicht.

Sie läßt sich vorteilhaft anwenden zur Erhöhung der Genauigkeit bei der Detektion von Lebewesen, vorzugsweise von Personen, die sich unbefugt in Gebäuden befinden oder aber bei der Erkennung der Identität von Lebewesen.

Beim erfindungsgemäßen elektronischen Lebensdetektionssystem ist die Empfangsschaltung so ausgebildet, daß sie eine Messung auch bei kleinsten Signal-Rausch-Verhältnissen sowie ohne Übersteuerungen ermöglicht.

Damit ist eine unverfälschte Erfassung der Modulations- (d.h. damit derzugrundeliegenden Körperschwingungs-) Frequenzen von sich im zu untersuchenden Bereich befindenden Lebewesen und damit - je nach Anwendungsbereich - auch deren Identifizierung und/oder die Feststellung ihres physischen Zustandes möglich.

Zu diesem Zweck ist vorteilhafterweise eine automatische Empfindlichkeitsregelung vorgeshen.

Weiterhin ist das System so ausgebildet, daß der Mikrowellensende-/-empfangseinrichtung eine Signalaufbereitungseinrichtung nachgeordnet ist, die das innerhalb der Empfangseinrichtung vor-aufbereitete Empfangssignal einer Frequenz- und/oder Korrelationsanalyse unterzieht.

Vorzugsweise verfügt das System - insbesondere bei Einsatz für Überwachungsaufgaben, bei denen eine Unterscheidung von Personen oder die Erkennung ihres aktuellen physischen zustandes erforderlich ist - über eine erste und eine zweite Speichervorrichtung zur Speicherung von Muster- bzw. Ist-Signalwerten und eine Vergleichereinheit zum Vergleichen der aus den Speichervorrichtungen entnommenen Signalmengen und zur Ausgabe eines das Vergleichsergebnis kennzeichnenden Signals an eine Anzeigevorrichtung.

Für die Durchführung des Datenvergleichs ist es von besonderem Vorteil, wenn die ermittelten, im Zeitbereich erfaßten Signale einer schnellen Fourier-Transformation (FFT) unterzogen werden. Die FFT-aufbereiteten Signale repräsentieren dabei jeweils ein Frequenzspektrum.

Die Genauigkeit der Detektion wird in vorteilhafter Weise dadurch erhöht, daß die Abtastung eines zu überwachenden Gebäudes durch Scannen im Sinne eines räumlichen Abtastens mit einem stark gebündelten Mikrowellen-Strahl mittels einer entsprechend ausgebildeten Richtantenne mit veränderlicher Ausrichtung erfolgt. Die Veränderung der Ausrichtung der Antenne wird dabei vorzugsweise elektrisch vorgenommen.

Nach einer bevorzugten Ausbildung der Erfindung weist ein elektronisches Überwachungssystem für die mobile Überwachung von einer Mehrzahl von Gebäuden zwecks Feststellung von Personen, die eines oder mehrere dieser Gebäude unbefugt betreten haben, erste und zweite Speichervorrichtungen, deren Speicheradressen Z₁ bis Zₙ den einzelnen zu überwachenden Gebäuden zugeordnet sind und deren Speicherinhalt aus Signalmengen zu gebäudespezifischen Merkmalen für die Normalsituation, objektbezogenen Sicherheitsmaßnahmen für den Fall einer unbefugten Benutzung eines Gebäudes, zeitlich variablen Belegungs- und Benutzungskriterien, Positionsdaten der Gebäude in dem jeweiligen Gelände sowie des Standpunkts eines Fahrzeugs zur Durchführung der mobilen Überwachnug und gebäudespezifischen Zusatzinformationen besteht, auf.

Die von dem mobilen Detektor des Überwachungssystem aufgenommenen, aus einem Gebäude reflektierten Mikrowellen werden nach Kompensation ihres unmodulierten Anteils und automatischer Pegeleinstellung einer schnellen Fourier-Analyse unterzogen und dann mit den in der Speichervorrichtung vorhandenen, dem gleichen Gebäude bzw. Gebäudeoder Geländeabschnitt zugeordneten Signalmengen verglichen.

In der Signalverarbeitungseinheit ist eine Einrichtung zur wahlweisen Akkumulation mehrerer Meßsignale vorgesehen, die in Betrieb gesetzt wird, falls das Signal-Rausch-Verhältnis einer Einzelmessung zur Gewinnung einer Fourier-Transformierten, die bezüglich des Rauschens mit der gespeicherten vergleichbar ist, nicht ausreicht.

Die Speichervorrichtungen sind zyklisch durch einen Multiplexer ansteuerbar ausgebildet. Die Inbetriebnahme der Sende- und Empfangs-Einrichtungen und das Auslesen der gebäudespezifischen Signalmengen aus den Speichervorrichtungen des Überwachungssystem erfolgt immer dann, wenn das für die mobile Überwachung eingesetzte Fahrzeug einen festgelegten und durch ein gesondertes Kontrollsystem überprüften Standort eingenommen hat.

Zur Auslösung eines Alarms wegen unbefugt in einem Gebäude vorhandener Personen ist es erforderlich, daß die Abweichung der gespeicherten (Muster)Signalmengen von den detektierten (Ist-)Signalmengen vorgegebenen Kriterien genügt bzw. - vereinfacht - einen bestimmten Betrag überschreitet.

Eine in vorteilhafter Weise nach einem Korrelations-Verfahren arbeitende Vergleichereinheit und eine geeignet dimensionierte Schwellwert-Stufe bewirken bei entsprechender Signalmengen-Abweichung die erforderliche Alarm-Auslösung, etwa auf einer Anzeigeeinrichtung. Für das Auslesen der Signaldaten, insbesondere der Positionsdaten für die einzelnen Gebäude aus der zweiten Speichervorrichtung ist ein Multiplexer vorgesehen, der von einem Zufallsgenerator angesteuert wird. Dadurch ist in vorteilhafter Weise eine Manipulation der Reihenfolge der Überwachung der einzelnen Objekte - und damit der Gestaltung einer Kontrollfahrt - vermeidbar und führt zu einer Erhöhung der Wirksamkeit der Überwachungsmaßnahmen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1a: ein Blockschaltbild einer bevorzugten Ausführungsform der Erfindung in schematisierter Darstellung,
- Figur 1b: die schematisierte Darstellung der in Figur 1a gezeigten Mikrowellensende-/-empfangsvorrichtung,
- Figur 1c: die schematisierte Darstellung der in Figur 1b gezeigten Signalverarbeitungsvorrichtung,
- Figur 2: die schematisierte Darstellung eines Details der in Figur 1 gezeigten Ausführungsform der Erfindung,
- Figur 3: die schematisierte Darstellung eines ersten Signalspeichers der Ausführungsform,
- Figur 4: die schematisierte Darstellung eines zweiten Signalspeichers der Ausführungsform,
- Figur 5: eine vorteilhafte Ausführung der Anzeigeeinrichtung entsprechend der Ausführungsform sowie
- Figur 6: eine Darstellung von detektierten Signaldaten, die aufgrund der Modulation reflektierter Mikrowellen-Anteile zum Nachweis von Personen oder Haustieren dienen.

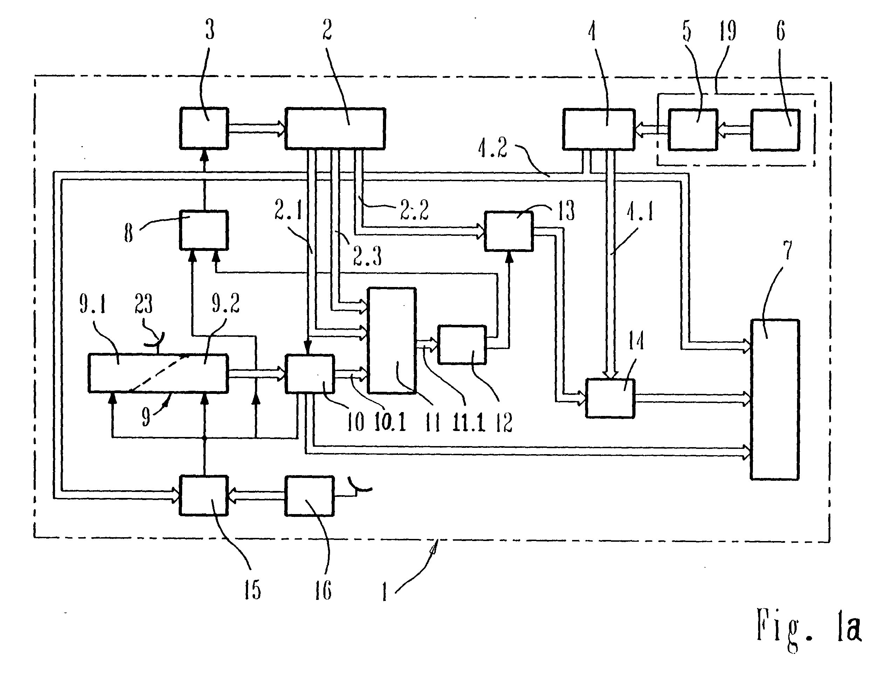

In Figur 1a ist ein Blockschaltbild eines Ausführungsbeispiels des elektronischen Überwachungssystems in schematisierter Form dargestellt, dessen wesentlichste Elemente eine Mikrowellensende-/-empfangsvorrichtung 9 mit einem Sender 9.1 und einem Empfänger 9.2, eine erste und eine zweite Speichervorrichtung 2 und 4, in denen die wichtigsten überwachungsspezifischen Datenmengen einzelner zu überwachender Gebäude gespeichert sind, und eine Vergleichereinheit 11 sind.

Der Sender 9.1 strahlt mittels einer Antenne 23 ein Überwachungssignal in den zu überwachenden Bereich, das dort teilweise reflektiert als Empfangssignal über die Antenne 23 dem Empfänger 9.2 zugeführt wird. Von dort gelangt es zu einer Aufbereitungseinheit, die daraus eine Ist-Signalmenge 10.1 gewinnt.

Die erste Speichervorrichtung 2 gibt Signalmengen 2.1 und 2.3 (Gebäudemerkmale unter Normalbedingungen und aktuelle Belegungskriterien für ein bestimmtes Gebäude Zₓ) an die Vergleichereinheit 11, der auch die Ist-Signalmenge 10.1 zugeleitet wird.

Die Vergleichereinheit ist so ausgebildet, daß sie einen Vergleich der Muster-Signalmengen mit der zugehörigen Ist-Signalmenge nach einem Korrelations-Verfahren anhand von aus den Signalmengen durch schnelle Fourier-Transformation gebildeten Frequenzspektren ausführt.

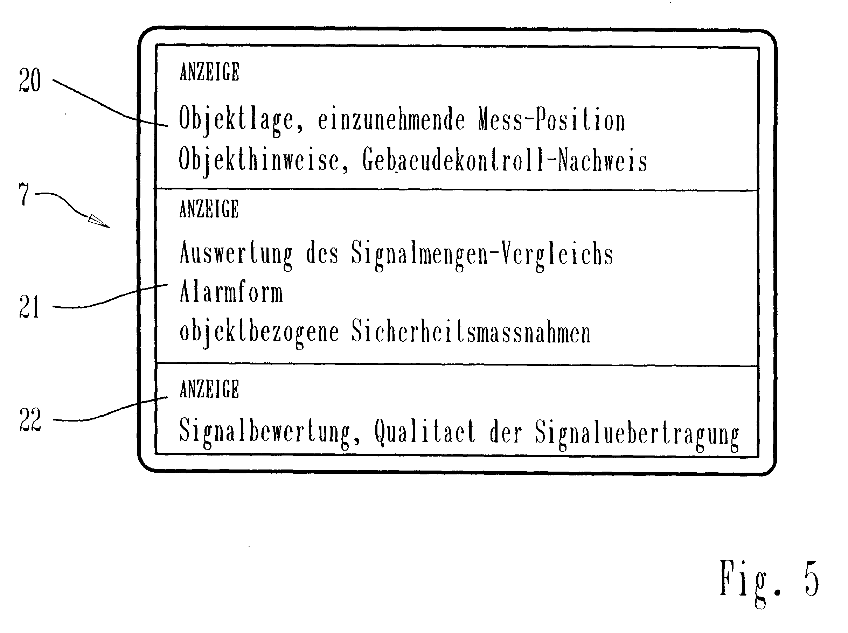

Das Vergleichsergebnis wird einem Schwellwert-Schalter 12 zugeführt. Die Überschreitung eines vorab zur Vermeidung von meßfehlerbedingtem Fehlalarm vorgegebenen Grades an zulässiger Abweichung zwischen den das zu überwachende Gebäude charakterisierenden Muster- und Ist-Signalmengen führt zur Aktivierung der Schaltstufe 13, von der über eine Summierschaltung 14 die Signalmengen 2.2 (objektbezogene Sicherheitsmaßnahmen aus der ersten Speichervorrichtung 2) und 4.1 (gebäudespezifische Zusatzinformationen aus der zweiten Speichervorrichtung 4) an die Alarmierungsvorrichtung bzw. Anzeigeeinheit 7 gelangen, wo eine Alarm-Auslösung unter gleichzeitiger Darstellung aller für den Diensttuenden relevanten Informationen (vgl. die Beschreibung zu Fig. 5) erfolgt.

Das Auslesen der Signalmengen aus den einzelnen Gebäuden Z₁ bis Zₙ zugeordneten Speicheradressen erfolgt aus beiden Speichervorrichtungen 2 und 4 jeweils durch eine externe Steuerung 3 bzw 19.

Die Steuerung 19 für die Speichervorrichtung 4 mit den Signalmengen 4.2 (Positionsdaten der zu überwachenden Gebäude) besteht aus einem zyklischen Multiplexer 5 und einem diesen ansteuernden Zufallsgenerator 6. Die Auswahl des ersten und der anschließend zu kontrollierenden Gebäude(s) erfolgt dadurch zufällig und in vorteilhafter Weise manipulationssicher.

Die Positionsdaten 4.2 werden auf der Anzeige-Einheit 7 zur Anzeige gebracht und gleichzeitig einer weiteren Vergleichereinheit 15 zugeführt, die mittels eines Navigations-Systems 16 der mobilen, in einem Fahrzeug installierten Mikrowellensende/Empfangs-Einrichtung den für die spezielle Überwachungsaufgabe vorgegebenen Standort zuweist.

Ist dieser Standort eingenommen, werden der Sender 9.1 und der Empfänger 9.2 eingeschaltet. Das Standort-Vergleichsergebnis wird gleichzeitig einer Torschaltung 8 zugeführt, die außerdem mit der Vergleichereinheit 11 verbunden ist. Werden durch die Vergleichereinheit die Erkennungs-Signalmengen des betreffenden Gebäudes verifiziert und ist die Mikrowellensende-/empfangseinrichtung 9 in Betrieb, so wird der zyklische Multiplexer 3 genau an der Speicheradresse Zₓ gestoppt, die dem gerade untersuchten Gebäude entspricht.

Damit kann bei einem bestimmten Grad der Abweichung zwischen den Muster-Signalmengen 2.1. 2.3 und der Ist-Signalmenge 10.1 zwecks Ausgabe einer Alarminformation die Signalmengen 2.2 (Sicherheitsmaßnahmen bei Feststellen unbefugter Benutzung des Gebäudes) zusammen mit den Positionsdaten und gebäudespezifischen Zusatzinformationen auf der Anzeige- bzw. Alarmierungsvorrichtung 7 dargestellt werden.

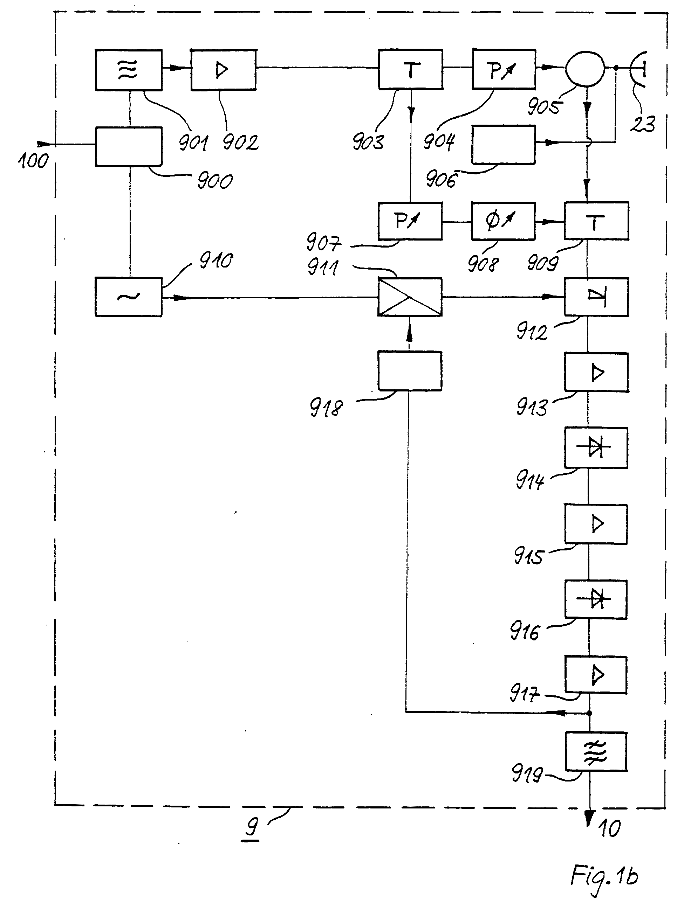

Figur 1b zeigt die Ausführung der Mikrowellensende-/-empfangseinrichtung 9 in Einzelheiten.

Die Einrichtung 9 wird durch eine Steuerung 900 kontrolliert. Ein Mikrowellengenerator 901 gibt auf entsprechenden Befehl der Steuerung 900 ein Mikrowellensignal mit einer Leistung von 20 mW aus, das einem Verstärker 902 zugeführt wird, der es auf eine Leistung von 200 mW verstärkt. Dessen Ausgang ist mit einem Richtungs- bzw. Spannungskoppler 903 verbunden, der einen Teil der Signalleistung abzweigt, während der Hauptteil über eine Leistungssteuerstufe 904 einem Zirkulator 905 und von diesem einer kombinierten Sende-/Empfangsantenne 23 zugeführt wird, über die die Mikrowellen in den zu überwachenden Bereich eingestrahlt werden.

Die Antenne weist eine ausgeprägte Richtcharakteristik auf und wird - in Abhängigkeit von der Größe des Bereiches und dem Abstand des Meßfahrzeuges zu diesem - mit einer an sich bekannten Antennensteuerung 906 schrittweise über einen zu überwachenden Raumbereich geführt. Die erforderlichen Daten werden der Antennensteuerung von der Vergleichereinheit 15 zugeführt, die sie auf die oben beschriebenen Weise erhält.

Die aus dem zu überwachenden Bereich reflektierten Mikrowellensignale werden von der Antenne 23 empfangen und zum Richtungs- bzw. Spannungskoppler 909 geleitet, wo dem Empfangssignal der im Richtungskoppler 903 entnommene und im Dämpfungsglied 907 sowie dem regelbaren Phasenschieber 908 in Amplitude und Phase so justierte Anteil des Sendesignals hinzugefügt wird, daß sich der unmodulierte Anteil des Empfangssignals und das abgezweigte Sendesignal auslöschen, so daß für die weitere Signalverarbeitung nur der modulierte Anteil des Empfangssignals erhalten bleibt.

Dieses Signal wird einem Modulator 912 zugeführt, wo es mit einer durch einen ebenfalls über die Steuerung 900 gesteuerten Tonfrequenzgenerator 910 erzeugten und über eine Addierstufe 911, deren Funktion weiter unten erläutert wird, zugeführten Modulationsspannung moduliert wird.

Über einen Mikrowellen-Vorverstärker 913, einen Demodulator 914, einen Schmalband-Verstärker 915, einen Gleichrichter 916 und einen NF-Verstärker 917 gelangt das Signal zu einem Verzweigungspunkt, von dem aus es einerseits über ein Bandfilter 919 schließlich der zweiten Signalaufbereitungseinheit 10, zum anderen über eine automatische Verstärkungsregelung 918 herkömmlicher Art der Addierstufe 911 zugeführt wird. Dort wird es der Modulationsspannung aufgeprägt, die dann dem Modulator 912 zugeführt wird.

Die zusätzliche Modulation des modulierten Anteils des Mikrowellen-Empfangssignals mit einer in ihrer Amplitude durch Rückkopplung aus dem Signal selbst gewonnenen Tonfrequenzspannung dient der automatischen Gewährleistung eines optimalen Arbeitsbereiches der Stufen 913 bis 919 und damit der Verbesserung des Signal-Rauschverhältnisses und der Verhinderung von Übersteuerungen im Signalaufbereitungsweg, die die gemessenen Frequenzspektren verfälschen und sich damit verheerend auf die Aussagefähigkeit der nachfolgend gewonnenen Ist-Signale auswirken würden.

Figur 1c zeigt einen schematisierten Aufbau der Signaverarbeitungseinheit 10 genauer. Die Einheit 10 wird durch eine Steuerung (CPU) 100 gesteuert, die außerdem die Steuerung 900 der Sende-/Empfangseinrichtung 9 steuert.

Das Signal vom Bandfilter 919 gelangt zu einem Spektrenanalysator 101 mit Prozeßrechner-Aufbau bzw. -Software, der auf bekannte Weise eine Frequenzanalyse durch schnelle Fourier-Transformation (FFT) des Signals zur Überführung vom Zeit- in den Frequenzbereich ausführt. Das transformierte Signal wird gleichzeitig in einem RAM 102 gespeichert, einem Display 104 zur visuellen Darstellung für den Bediener und einem Vergleicher 103 zugeführt. Das Display 104 kann dabei auch mit der Anzeigevorrichtung 7 identisch sein.

Bei einer auf Rettungsaufgaben zugeschnittenen Ausführungsform ist im übrigen vorteilhafterweise der/den Anzeigevorrichtung(en) des Lebensdetektionssystems auch ein akustischer und/oder deutliche Signalwirkung entfaltender optischer Anzeiger für den Empfang modulierter Signale - d. H. den erfolgten Nachweis lebender Personen - unabhängig von deren genauerer Auswertung zugeordnet. Das Ansteuersignal für einen solchen Signalgeber kann nach dem Bandfilter 919 oder nach dem Spektrenanalysator 101 abgegriffen werden.

Der Vergleicher 103 erhält außerdem von einem Eichsignalgeber 105 ein Signal, dessen Signal-Rausch-Verhältnis einem für eine korrekte Weiterverarbeitung im Vergleicher 11 erforderlichen Wert entspricht. Dieses Signal wird z. B. aus in der Speichervorrichtung 2 gespeicherten Muster-Signalen 2.3 gewonnen.

Ist das Signal-Rausch-Verhältnis des Meßsignals kleiner als das des Eichsignals bzw. als ein vorgegebener Mindestwert, so gibt der Vergleicher ein diesen Umstand kennzeichnendes Signal an die CPU aus, die daraufhin die Steuerung 900 der Sende-/Empfangseinrichtung anweist, einen weiteren Meßvorgang auszuführen. Das in dessen Ergebnis erhaltene Meßsignal gelangt wiederum in den Spektrenanalysator 101, den Speicher 102 (wo es an einem anderen Speicherplatz abgelegt wird als das erste Meßsignal) und das Display 104. Es gelangt jedoch auf Veranlassung der CPU 100 nicht direkt zum Vergleicher 103, sondern wird gemeinsam mit dem ersten Meßsignal aus dem Speicher 102 einem an sich bekannten Spektren-Akkumulator 106 zugeführt, wo es mit dem ersten Meßsignal überlagert und dadurch das Signal-Rausch-Verhältnis verbessert wird. Das im Spektren-Akkumulator 106 gewonnene Spektrum wird dem Vergleicher zugeführt und daraufhin geprüft, ob es das erforderliche Signal-/Rausch-Verhältnis aufweist. Ist dies der Fall, weist die CPU 100 den Vergleicher 100 zur Ausgabe des Spektrums an den Vergleicher 11 und die Steuerung 900 zum Abwarten auf einer neuen Steuerbefehl an. Reicht das Signal-/Rausch-Verhältnis noch nicht: aus, so wird die Messung solange wiederholt und das Meßergebnis akkumuliert, bis der benötigte Wert erreicht ist oder abgebrochen wird.

Zum Abbruch des Meßvorganges sowie zur Eingabe von die Signalverarbeitung steuernden Befehlen des Bedieners dient eine Eingabevorrichtung 107.

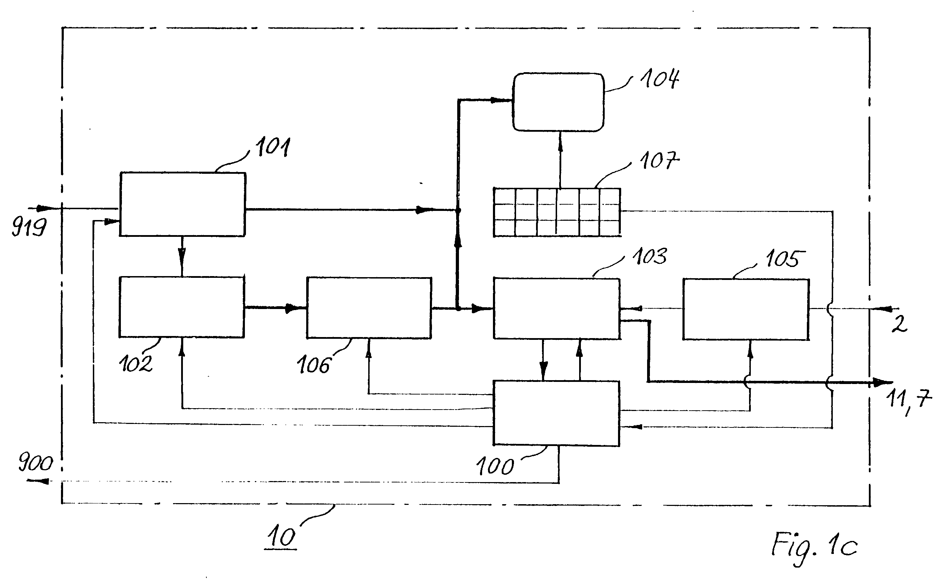

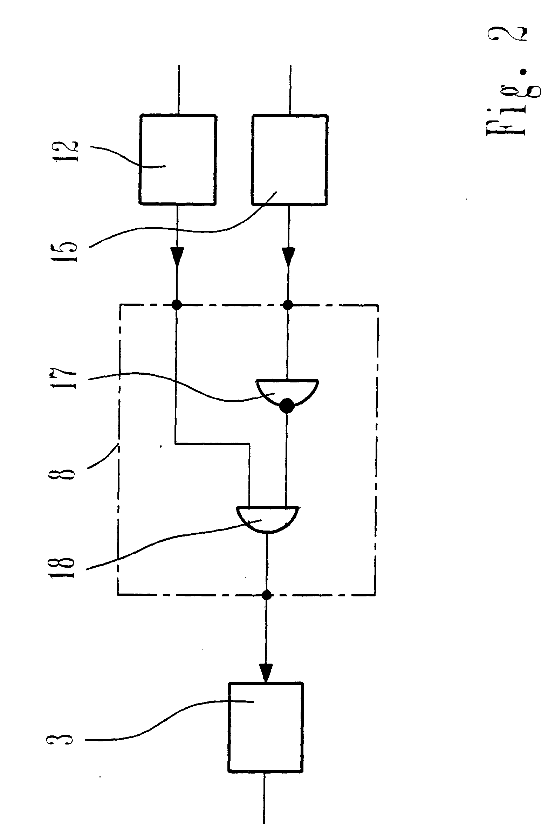

Figur 2 zeigt eine vorteilhafte Ausführungsform der in Figur 1 beschriebenen Torschaltung 8.

Die Torschaltung 8 enthält ein UND-Gatter 18, dessen Eingänge zum einen direkt mit der Schwellwert-Stufe 12 der ersten Vergleichereinheit (Bezugsziffer 11 in Figur 1) und zum anderen über einen Negator 17 mit der Vergleichereinheit 15 für die Kontrolle der Meß-Standorte verbunden sind.

Ist der richtige Standort für die Messung eingenommen, d. h. stimmen Muster und Ist-Erkennungs-Signalmengen des zu untersuchenden Raumbereiches überein, so schaltet das UND-Gatter 18 durch, und der zyklische Multiplexer 3 stoppt an der dem aktuell zu überwachenden Raumbereich zugeordneten Speicherstelle Zₓ der ersten Speichervorrichtung (Bezugsziffer 2 in Figur 1), deren Inhalt zur Anzeige gebracht werden kann.

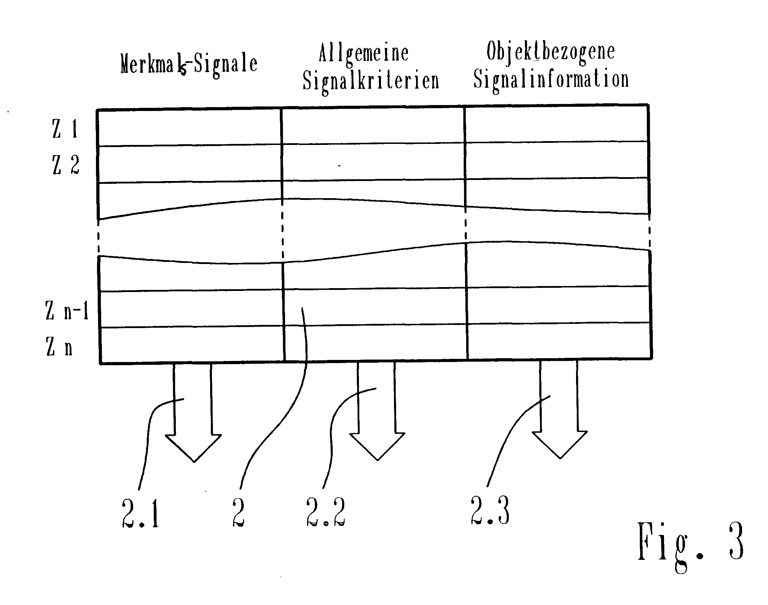

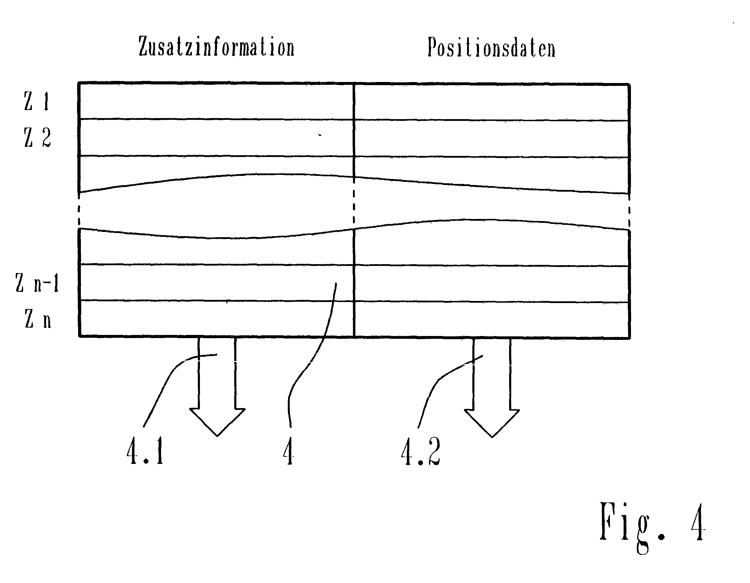

In den Figuren 3 und 4 ist in schematisierter Form der Aufbau der als Matrix-Speicher ausgebildeten ersten und zweiten Speichervorrichtung 2 und 4 dargestellt. Für einen Überwachungsbereich von n Gebäuden enthält jeder der beiden Matrixspeicher n Zeilen, die mit Z₁ bis Zₙ bezeichnet und jeweils einem der Gebäude zugeordnet sind. Die Signalmengen 2.1, 2.2 und 2.3 beziehen sich - in dieser Reihenfolge - auf gebäudespezifische Merkmale (Merkmals-Signale), objektbezogene Sicherheitsmaßnahmen (allgemeine Signalkriterien) und Körperschwingungsspektren aller Personen oder Haustiere, die sich normalerweise in dem Gebäude befinden. Der Matrix-Speicher 4 enthält ebenfalls zeilenweise den einzelnen Gebäuden zugeordnet die Positionsdaten 4.2 sowie Zusatzinformationen 4.1, durch die beispielsweise Kontrollzeiten vorgegeben werden können.

Eine günstige Form der Darstellung des Überwachungsergebnisses sowie - im Alarmfalle - von Alarminformationen ist in Figur 5 als Display der Anzeige-Einheit 7 dargestellt.

Das Display ist in drei Anzeigebereiche 20, 21, 22 aufgeteilt, in denen jeweils verbale Informationen dargestellt sind. Der obere Bereich 20 ist für eine Anzeige der Gebäudelage und der einzunehmenden Meß-Standorte vorgesehen. Gleichzeitig wird ein Kontrollvermerk über die jeweils durchgeführte Überwachung angezeigt (und gespeichert). Im mittleren Anzeigebereich 21 erscheint im gegebenen Fall im Ergebnis der Auswertung der Muster- mit den Ist-Signalmengen eine Alarmanzeige sowie eine Darstellung relevanter Sicherheits- oder auch Rettungsmaßnahmen, die im Alarmfall unter Beachtung gebäudespezifischer Besonderheiten durchzuführen sind. Das dritte Displayfeld 22 dient der Anzeige der Qualität der Signalübertragung und damit der Information über das Erfordernis, gegebenenfalls eine Wiederholung des Überwachungsvorgangs vorzunehmen.

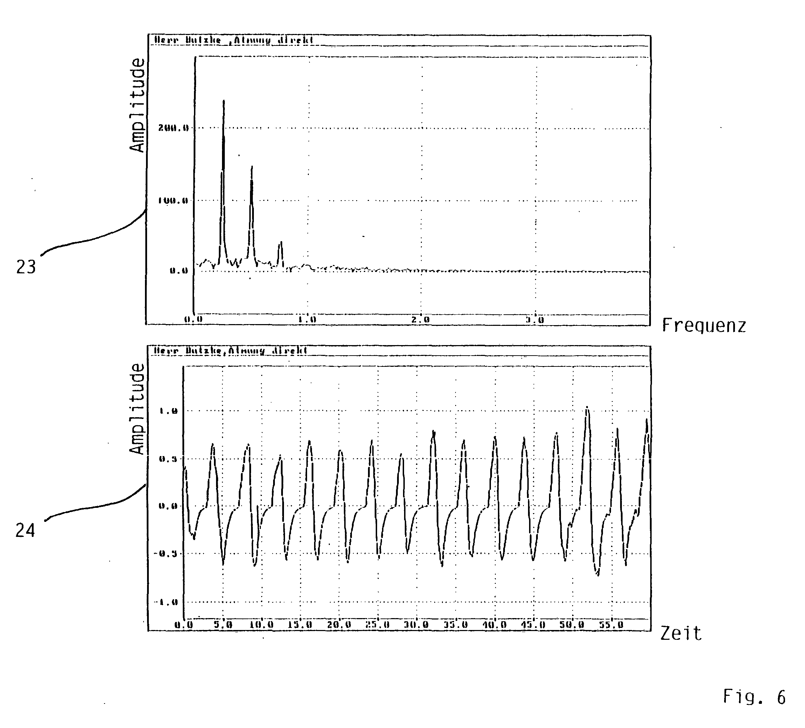

In Figur 6 ist zur Veranschaulichung des Meßprinzips in Diagrammform das Meßergebnis einer direkten Mikrowellen-Detektion der Atmung einer Person dargestellt. Das Amplituden-Zeit-Diagramm 24 zeigt den normalen Atemrythmus einer männlichen Person für den Zeitbereich von einer Minute. Das aus diesem Kurvenverlauf mittels einer Fourier-Transformation abgeleitete Frequenzspektrum 23 zeigt im ersten Viertel der dargestellten normierten Frequenzbereichs drei charakteristische Peaks, deren Frequenzlage und Amplitudenwert personenspezifisch sind.

Eine verfeinerte Meßtechnik erlaubt auf dieser Grundlage die Ausfertigung von individuellen, fingerabdruckähnlichen Körperschwingungsbildern, deren charakteristische Merkmale - insbesondere Frequenzanteile aus Oberschwingungen - die Identifizierung einer Person sogar unabhängig von deren aktuellem Atem- und Herzrhythmus erlauben. Auf dieser Grundlage enthält dann die etwa einer Überwachung zugrundegelegte Muster-Signalmenge die Körperschwingungsbilder aller Personen und/oder Tiere, die sich in einem fraglichen Gebäude oder Gebäudeabschnitt berechtigt aufhalten. Die Muster-Signalmenge kann im Rahmen einer Referenzmessung, bei der sich alle berechtigten Personen im Gebäude befinden, zusammenhängend bestimmt oder auch aus einzelnen, getrennt aufgenommenen Körperschwingungsspektren berechtigter Personen synthetisiert werden. Treten in der gemessenen Ist-Signalmenge Frequenzbilder auf, die für die befugten Personen atypisch sind, bedeutet dies die Anwesenheit Unbefugter.

Ähnlich lassen sich aus den Körperschwingungsspektren Aussagen über den physischen Zustand detektierter Personen - etwa von Lawinen- oder Erdbebenopfern - bereits vor deren Bergung gewinnen, was eine präzise Bestimmung der erforderlichen Rettungsmaßnahmen ermöglicht.

Ein meßtechnisch weniger verfeinertes Überwachungssystem im Rahmen der Erfindung umfaßt die Zuweisung eines Kennsignalgebers an jede sich in einem zu überwachenden Abschnitt befugt aufhaltenden Person, ggf. auch an Haustiere. Ein solcher Kennsignalgeber gibt ein mittels der Mikrowellenabtastung des fraglichen Bereiches erfaßbares Kennsignal ab, das zusammen mit den Körperschwingungen der sich befugt in diesem Bereich aufhaltenden Personen und/oder Tiere nachgewiesen wird und diese im Frequenzbild als Befugte kennzeichnet. Tauchen nun im Frequenzbild Anteile ohne Kennsignalzuordnung auf, so sind diese einem Unbefugten zuzuordnen und führen zur Auslösung eines Alarms. In einer speziellen Ausgestaltung ist der Kennsignalgeber ein - zweckmäßigerweise in seiner Frequenz veränderbarer - Infraschallsender. der ein charakteristisches Frequenzpeak in der Nähe der Körperschwingungs-Grundfrequenzen liefert, das durch den Mikrowellenempfänger leicht nachweisbar ist.

Bei dieser letzteren Variante brauchen von den befugten Personen und/oder Tieren keine individuellen Körperschwingungsbilder aufgenommen und ausgewertet zu werden, sondern es genügt eine im wesentlichen im Bereich der Grundschwingungen betriebene Meßvorrichtung.

Eine weitere Ausbildung der Erfindung besteht darin, daß das Überwachungssystem nicht mobil, sondern ortsfest installiert ist. Dabei entfallen einige der im obigen Ausführungsbeispiel beschriebenen Funktionsgruppen und Verfahrensschritte, insbesondere die mit der korrekten Lokalisierung des zu überwachenden Gebäudes und des Meßfahrzeuges verbundenen.

In speziellen Ausführungen kann auch auf eine Richtantenne und deren Steuerung verzichtet und das Meßsignal über einfache, ggf. sogar ortsfest installierte Metallflächen abgestrahlt und aufgenommen werden.

## Patentansprüche

1. Elektronisches Lebensdetektionssystem mittels durch ein Lebewesen reflektierter und durch dessen Körperschwingungen modulierter Mikrowellen, mit einer Mikrowellensende-/-empfangseinrichtung (9) zur Erzeugung und Einstrahlung der Mikrowellen sowie zum Empfang und zur Aufbereitung des Reflexionssignals unter Elimination des im Empfangssignal enthaltenen unmoduliert reflektierten Signals durch eine erste Signalaufbereitungseinrichtung sowie einer Anzeigevorrichtung (7) zur Ausgabe des Indikations-Ergebnisses,
**gekennzeichnet durch** eine der Mikrowellensende-/-empfangseinrichtung (9) nachgeordnete zweite Signalaufbereitungseinrichtung (10), die das **durch** die erste Signalaufbereitungseinrichtung (903 bis 919) verarbeitete Mikrowellensignal einer Frequenzanalyse des körperschwingungsmodulierten Signals unterzieht, und Mittel zum Vergleich mit gespeicherten Mustern zur Identifizierung des Lebewesens und/oder Feststellung seines physischen Zustandes, wobei die Mikrowellensende/-empfangseinrichtung (9) für einen Frequenzbereich von 1,3 bis 1,6 GHz ausgelegt ist.

2. System nach Anspruch 1 oder 2,
**dadurch gekennzeichnet, daß** die zweite Signalaufbereitungseinrichtung (10) eine Verarbeitungseinheit (101) zur Ausführung einer schnellen Fourier-Transformation und/oder Korrelationsanalyse bezüglich des modulierten Anteils des Empfangssignals aufweist.

3. System nach einem der vorangehenden Ansprüche,
**dadurch gekennzeichnet, daß** die erste Signalaufbereitungseinrichtung (903 bis 919) eine Kompensationsschleife (903 bis 909) und eine Regelschleife (910 bis 918) zur Amplitudenregelung eines modulierten Empfangssignal-Anteils aufweist.

4. System nach einem der Ansprüche 1 bis 3,
**dadurch gekennzeichnet, daß** die zweite Signalaufbereitungseinrichtung (10) eine Einrichtung (100,102,103,105,106) zur wahlweisen Signalakkumulation mehrerer nacheinander aufgenommener Empfangssignale zwecks Verbesserung des Signal-Rausch-Verhältnisses aufweist.

5. System nach einem der vorangehenden Ansprüche,
**dadurch gekennzeichnet**, daßdie Eliminationsvorrichtung aus einer Kompensationsschleife (903 bis 909) besteht, mittels der dem empfangenen Signal das unmodulierte Sendesignal verzögert und gegenphasig zugeführt wird.

6. System nach einem der vorangehenden Ansprüche,
**dadurch gekennzeichnet, daß** die erste Signalaufbereitungseinrichtung einen Generator (910) zur Erzeugung einer Modulationsspannung, eine Baugruppen (913 bis 917) zur Verstärkung, Demodulation und Filterung des modulierten Anteils des Empfangssignals nachgeordnete automatische Verstärkungsregelung (918), eine Addierstufe (911) zum Mischen der Ausgangssignale des Generators (910) und der Verstärkungsregelung (918) und einen den Baugruppen (913 bis 917) zur Verstärkung, Demodulation und Filterung vorgeschalteten Modulator (912) zur zusätzlichen Modulation des modulierten Anteils des Empfangssignals mit dem Ausgangssignal der Addierstufe (911) aufweist.

7. System nach einem der Ansprüche 4 bis 6,
**dadurch gekennzeichnet, daß** die Einrichtung zur Signalakkumulation eine zweite Speichervorrichtung (102) zur Speicherung nacheinander aufgenommener, aufbereiteter Meßsignale, einen Eichsignalgeber (105) zur Lieferung eines bezüglich des Signal-Rausch-Verhältnisses als Normal dienenden, vorab gespeicherten Signals, eine Addierstufe (106) zur Akkumulation nacheinander aufgenommener, in der zweiten Speichervorrichtung (102) gespeicherter Meßsignale, eine zweite Vergleichereinheit (103) zum Vergleich eines aufbereiteten Meßsignals oder eines Ausgangssignals der Addierstufe (106) mit dem Eichsignal sowie eine Steuerung (100) zur Steuerung des Zusammenwirkens der zweiten Speichervorrichtung (102), des Eichsignalgebers (105), der Addierstufe (106) und der zweiten Vergleichereinheit (103) sowie der Mikrowellensende-/-empfangseinrichtung (9) aufweist.

8. System nach einem der vorangehenden Ansprüche,
**gekennzeichnet durch**
- eine erste Speichervorrichtung (2) zur Speicherung und Ausgabe mindestens einer Muster-Signalmenge (2.1,2.3), die einen als Normalzustand der Abwesenheit bestimmter Lebewesen in dem zu untersuchenden Bereich dienender Zustand beschreibt, und
- eine erste Vergleichereinheit (11) zum Vergleich einer **durch** die zweite Signalaufbereitungseinrichtung (10) gelieferten Ist-Signalmenge (10.1) und der aus der ersten Speichervorrichtung (2) ausgegebenen mindestens einen Muster-Signalmenge (2.1, 2.3) miteinander, die eine die Abweichung der Ist- von der Muster-Signalmenge kennzeichnende Ausgangswertmenge (11.1) ausgibt.

9. System nach einem der vorangehenden Ansprüche,
**dadurch gekennzeichnet, daß** die Vergleichereinheit (11) eine Einrichtung zur Verarbeitung der Ist-Signalmenge (10.1) und der mindestens einen Muster-Signalmenge (2.1, 2.3) nach einem Korrelationsverfahren aufweist.

10. System nach einem der vorangehenden Ansprüche,
**dadurch gekennzeichnet, daß** die Antenne (23) der Mikrowellensende-/-empfangseinrichtung (9) eine ausgeprägte Richtcharakteristik aufweist sowie horizontal und/oder vertikal, insbesondere schrittweise, schwenkbar ist.

## Claims

1. An electronic life detection system by means of microwaves reflected through a life form and modulated through the body oscillations of which, having a microwave transmitting/receiving device (9) for producing and radiating microwaves likewise for receiving and for the preparation of the reflection signal with elimination of the unmodulated reflected signal obtained in the receiving signal, through a first signal preparation device likewise an indicator device (7) for issuing the indicator results,
**characterised by** a second signal preparation device (10) arranged after the microwave transmitting/receiving device (9), which subjects the microwave signal processed through the first signal preparation device (903-919) to a frequency analysis of the body-oscillation modulating signal, and means for comparison with stored patterns for identifying the life form and/or for establishing its physical state, and where the microwave transmitting/receiving device (9) is designed for a frequency range of 1,3-1,6 GHz.

2. A system according to claim 1 or 2, **characterised in that** the second signal preparation device (10) has a processing unit (101) for carrying out a fast Fourier transformation and/or a correlation analysis in relation to the modulated part of the receiving signal.

3. A system according to any of the above claims, **characterised in that** the first signal preparation device (903-919) has a compensating loop (903-909) and a loop (910-918) for the amplitude control of a modulated part of receiving signal.

4. A system according to any of claims 1-3, **characterised in that** second signal preparation device (10) has a device (100,102,103,105,106) for the selective signal accumulation of a plurality of successively received receiving signals for the purpose of improving the signal-noise-ratio.

5. A system according to any of the above claims, **characterised in that** the elimination device is comprised of a compensating loop (903-909), by means of which the unmodulated transmitting signal is supplied to the receiving signal delayed and in opposition of phase.

6. A system according to any of the above claims, **characterised in that** the first signal preparation device has a generator (910) for producing a modulation voltage, an assembly (913-917) for amplification, demodulation and filtering of the modulated part of a receiving signal arranged after automatic gain control (918), an adding stage (911) for mixing the output signal of generator (910) and gain control (918) and a modulator (912) connected in series to assembly (913-917) for amplification, demodulation and filtering, for additional modulation of the modulated part of the receiving signal with the output signal of adding stage (911).

7. A system according to any of claims 4-6, **characterised in that** the device for signal accumulation has a second storage device (102) for storing successively received, prepared measuring signals, an adjustment signal transmitter (105) for supplying a signal first stored, serving as normal in relation to the signal-noise-ratio, an adding stage (106) for the accumulation of successively received measuring signals stored in the second storage device (102), a second comparison unit (103) for comparing a prepared measuring signal or an adding stage (106) output signal with the adjustment signal likewise a control unit (100) for controlling the co-operation of second storage device (102) of adjustment signal transmitter (105), adding stage (106) and second comparison unit (103) likewise the microwave transmitting/receiving device (9).

8. A system according to any of the above claims, **characterised by** a first storage device (2) for storage and output of at least a pattern-signal amount (2.1,2.3), which describes a state serving as a normal state for the presence of specific life forms in the area to be monitored, and a first comparison unit (11) for an actual signal amount (10.1) supplied via the second signal preparation device (10), and at least the one pattern-signal amount (2.1,2.3) emitted from the first storage device (2) with one another, which outputs an initial value amount (11.1) characterising the variation of the actual- from the pattern- signal amount.

9. A system according to any of the above claims, **characterised in that** the comparison unit (11) has a device for processing the actual signal amount (10.1) and which has at least a pattern signal amount (2.1,2.3) after a correlation process.

10. A system according to any of the above claims, **characterised in that** antenna (23) of the microwave transmitting/receiving device (9) has a distinct directional characteristic and is likewise horizontally and/or vertically pivotable, and in particular step-wise.

## Revendications

1. Système électronique de détection de vie à l'aide de micro-ondes réfléchies par un être vivant et modulées par les vibrations du corps de ce dernier, comportant un dispositif d'émission/réception de micro-ondes (9) pour produire et rayonner les micro-ondes ainsi que recevoir et traiter le signal de réflexion moyennant l'élimination du signal réfléchi non modulé, continu dans le signal de réception, au moyen d'un premier dispositif de traitement de signaux ainsi qu'un dispositif d'affichage (7) pour la délivrance du résultat de l'indication, **caractérisé par** un second dispositif de traitement de signaux (10), qui est disposé en aval du dispositif d'émission/réception de micro-ondes (9) et qui soumet le signal de micro-ondes traité par le premier dispositif de traitement de signaux (903 à 919), à une analyse de fréquences du signal modulé par des vibrations du corps, et des moyens pour établir une comparaison avec les modèles mémorisés pour l'identification de l'être vivant et/ou la détermination de son état physique, le dispositif d'émission/réception de micro-ondes (9) étant conçu pour une gamme de fréquences de 1,3 à 1,6 GHz.

2. Système selon la revendication 1 ou 2, **caractérisé en ce que** le second dispositif de traitement de signaux (10) est une unité de traitement (101) servant à exécuter une transformation de Fourier rapide et/ou une analyse de corrélation concernant le pourcentage modulé du signal de réception.

3. Système selon l'une des revendications précédentes, **caractérisé en ce que** le premier dispositif de traitement de signaux (903 à 919) possède une boucle de compensation (903 à 909) et une boucle de régulation (910 à 918) servant à régler l'amplitude d'une composante modulée du signal de réception.

4. Système selon l'une des revendications 1 à 3, **caractérisé en ce que** le second dispositif de traitement de signaux (10) comporte un dispositif (100,102,103,105, 106) servant à cumuler au choix plusieurs signaux de réception enregistrés successivement, en vue d'améliorer le rapport signal/bruit.

5. Système selon l'une des revendications précédentes, **caractérisé en ce que** le dispositif d'élimination est constitué par une boucle de compensation (903 à 909), au moyen de laquelle le signal d'émission non modulé est appliqué d'une manière retardée et en opposition de phase, au signal reçu.

6. Système selon l'une des revendications précédentes, **caractérisé en ce que** le premier dispositif de préparation de signaux comporte un générateur (910) pour produire une tension de modulation, une unité de régulation d'amplification automatique (918) installée en aval de modules (913 à 917) pour amplifier, démoduler et filtrer la composante modulée, un étage additionneur (911) pour mélanger les signaux de sortie du générateur (910) et de l'unité de régulation d'amplification (918) et un modulateur (912) branché en amont des modules (913 à 917) pour l'amplification, la démodulation et le filtrage et servant à appliquer une modulation supplémentaire à la composante modulée du signal de réception du signal de sortie de l'étage additionneur (911).

7. Système selon l'une des revendications 4 à 6, **caractérisé en ce que** le dispositif d'accumulation du signal comporte un second dispositif de mémoire (122) pour mémoriser des signaux de mesure préparés et enregistrés successivement, un générateur de signal d'étalonnage (105) servant à délivrer un signal préalablement mémorisé, utilisé comme étalon par rapport au rapport signal/bruit, un étage additionneur (106) pour accumuler des signaux de mesure enregistrés successivement et mémorisés dans le second dispositif de mémoire (102), une seconde unité formant comparateur (103) pour comparer un signal de mesure préparé à un signal de sortie de l'étage additionneur (106) au signal d'étalonnage, ainsi qu'une unité de commande (100) pour commander la coopération du second dispositif de mémoire (102), du générateur de signal d'étalonnage (105), de l'étage additionneur (106) et de la seconde unité formant comparateur (103) ainsi que du dispositif d'émission/réception de micro-ondes (9).

8. Système selon l'une des revendications précédentes, **caractérisé par**
- un premier dispositif de mémoire (2) pour mémoriser et délivrer au moins une quantité (2.1, 2.3) du signal de modèle, qui décrit un état utilisé comme état normal de la présence d'êtres vivants déterminés, dans la zone à examiner, et
- une première unité formant comparateur (11) servant à comparer entre elles une quantité de signal réel (10.1) délivré par le second dispositif de traitement de signaux (10), et la au moins une quantité de signal de modèle (2.1, 2.3) délivrée par le premier dispositif de mémoire (2), et qui délivre une valeur quantitative de sortie (11.1), caractérisant l'écart entre la quantité de signal réel et la quantité de signal du modèle.

9. Système selon l'une des revendications précédentes, **caractérisé en ce que** l'unité formant comparateur (11) comporte un dispositif pour traiter la quantité de signal réelle (10.1) et la au moins une quantité de signal de modèle (2.1, 2.3) selon un procédé de corrélation.

10. Système selon l'une des revendications précédentes, **caractérisé en ce que** l'antenne (23) du dispositif d'émission/réception de micro-ondes (9) possède une caractéristique directionnelle accusée, et peut pivoter horizontalement et/ou verticalement, notamment pas-à-pas.
